# EUROPEAN PATENT SPECIFICATION

(11) **EP 1 734 940 B1**
(45) Date of publication and mention of the grant of the patent: **08.10.2008**
(21) Application number: 04720093.6
(22) Date of filing: 12.03.2004
(51) Int. Cl.: A61K 31/138, A61K 31/485, A61P 25/00, A61P 25/04

(54) **COMBINATIONS OF DERAMCICLANE AND OPIOIDS FOR USE AS ANALGESICS**
ZUSAMMENSETZUNGEN AUS DERAMCICLAN UND OPIOIDEN ZUR VERWENDUNG ALS SCHMERZMITTEL
COMBINAISONS DE DERAMCICLANE ET D'OPIOIDES DESTINES A L'UTILISATION ANALGESIQUE

(43) Date of publication of application: 27.12.2006
(73) Proprietor: EGIS Gyógyszergyár Nyrt, 1106 Budapest (HU)
(72) Inventor: GACSÁLYI, István, H-1021 Budapest (HU); GIGLER, Gábor, H-1119 Budapest (HU); HÁRSING, László, Gábor, H-1071 Budapest (HU); LÉVAY, György, H-2092 Budakeszi (HU); SZÉNÁSI, Gábor, H-1031 Budapest (HU)
(74) Representative: Beszédes, Stephan G.
(86) International application number: PCT/HU2004/000021
(87) International publication number: WO 2005/087213

(56) References cited:
- EP-A- 1 052 243
- WO-A-98/17230
- WO-A-03/007926
- US-A- 5 652 270

## Description

The invention relates to a combined analgesic pharmaceutical composition.

### TECHNICAL BACKGROUND

(1R,2S,4R)-(-)-2-[N,N-(dimethylaminoethoxy)]-2-phenyl-1,7,7-trimethylbicyclo[2.2.1]heptane of the Formula 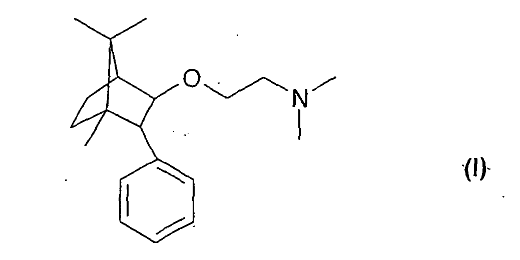 (International Non-Proprietory Name: deramciclane) is an anxiolytic pharmaceutical active ingredient which falls under the general Formula of HU 179,164. The preparation of deramciclane is described in HU 212,574.

Deramciclane showed considerable effects in different animal models of anxiety and stress. In the Vogel punished drinking test deramciclane was active in 1 and 10 mg/kg after oral administration *[*Gacsályi et. al, Receptor binding profile and anxiolytic activity of deramciclane (EGIS-3886) in animal models, Drug Dev. Res. 40: p. 338-348, (1997*)].* In the social interaction model, the compound increased the time spent with social interactions after the single 0.7 mg/kg oral treatment. In the light-dark model, [Crawley, J.N. Neuropharmacological specifity of a simple model of anxiety for the behavioural actions of benzodiazepine, Pharmacol. Biochem. Behavior, 15: p. 695-699 (1981*)*] deramciclane proved to be active in a single oral dose of 3 mg/kg sc. In the marble burying model *[*Broekkamp, C.L. et al, Major Tranquillizers Can Be Distinguished from Minor Tranquillisers on the Basis of Effects on Marble Burying and Swim-Induced Grooming in Mice. Eur. J Pharmacol. 126: p. 223-229, (1986*)]* the molecule was active in 10 and 30 mg/kg after oral treatment. Deramciclane did not possess analgesic activity in the above mentioned doses.

Additionally to its analgesic potential morphine, unfortunately, has numerous side effects which limit its therapeutic use. These side effects are tolerance, psychic and physiological dependence, and sometimes, fatal depression of respiration.

The object of the present invention is to develop a medical product which potentiates the analgesic effect of morphine.

Further object of the present invention is to develop a pharmaceutical product having analgesic effect.

The above objects are solved by the development of the combined medical product of the present invention.

The invention is based on the recognition that (1R,2S,4R)-(-)-2-[N,N-(dimethylaminoethoxy)]-2-phenyl-1,7,7-trimethyl-bicyclo[2.2.1]heptane of the Formula I and pharmaceutically acceptable acid addition salts thereof potentiate the analgesic effect of morphine.

Further basis of the present invention is the recognition that the combination of (1R,2S,4R)-(-)-2-[N,N-(dimethylaminoethoxy)]-2-phenyl-1,7,7-trimethylbicyclo[2.2.1]heptane of the Formula I and pharmaceutically acceptable acid addition salts thereof with morphine, opioide type of analgesics and/or other type of analgesics have outstanding analgesic efficacy.

The morphine potentiating effect of deramciclane, from the therapeutical point of view, is very advantageous because by this means the effective dose of morphine can be reduced resulting in the occurrence of less unwanted side effects.

The combination of deramciclane with low doses of morphine is beneficial in the following respects:
1. One will be able to successfully use morphine in low doses in which no effect and consequently no side effects occur (Table 1). In combination with deramciclane, the analgesic effect occurred and has been potentiated without abuse potential (deramciclane has no abuse potential).
2. If one uses the doses of morphine in which the analgesic effects are expressed (e.g. in cancer therapy), deramciclane will further potentiate the analgesic effect of morphine. This means that to achieve the same analgesic effect one can use lower doses of morphine with less side effects.
3. In patients where morphine tolerance has already been developed, in combination with deramciclane the doses of morphine could be decreased and/or not increased further.

The present invention relates to combined pharmaceutical compositions for treating pain.

### SUMMARY OF THE INVENTION

According to the present invention there is provided a combined analgesic pharmaceutical composition which comprises as component A) (1R,2S,4R)-(-)-2-[N,N-(dimethylaminoethoxy)]-2-phenyl-1,7,7-trimethylbicyclo[2.2.1]heptane or a pharmaceutically acceptable acid addition salt thereof and as component B) morphine, an opioide type analgesic and/or a non-opioide type analgesic in admixture with suitable pharmaceutical carriers and/or auxiliary agents.

### DETAILED DESCRIPTION OF THE INVENTION

The combined analgesic pharmaceutical composition according to the present invention comprises as component A) (1R,2S,4R)-(-)-2-[N,N-(dimethylaminoethoxy)]-2-phenyl-1,7,7-trimethylbicyclo[2.2.1]heptane of the Formula I or a pharmaceutically acceptable acid addition salt thereof which is described in Hungarian patent application HU 1559/99.

The combined analgesic pharmaceutical composition according to the present invention comprises as component B) an opioide type analgesic selected from tramadol, codeine, dihydrocodeine, nalbufine or buprenorfine or a pharmaceutically acceptable salt thereof.

The combined analgesic pharmaceutical composition according to the present invention comprises as component B) a non-opioide type analgesic selected from acetyl salicylic acid, paracetamol, aminophenazone, diclofenac, naproxen, ibuprofene, piroxicam or a pharmaceutically acceptable salt thereof.

The combined analgesic pharmaceutical composition according to the present invention comprises component A) and as component B) morphine or an opioide type analgesic in a weight ratio of 500:1, preferably 100:1, particularly preferably 30:1.

The combined analgesic pharmaceutical composition according to the present invention comprises component A) and as component B) a non-opioide type analgesic in a weight ratio of 1:500, preferably 1:100, particularly preferably 1:30.

The daily dosage of the combined analgesic pharmaceutical composition according to the present invention is 0.1-150 mg/kg, preferably 1-150 mg/kg, particularly preferably 10-150 mg/kg.

The term "pharmaceutically acceptable acid addition salt" relates to salts formed with pharmaceutically acceptable inorganic or organic acids. For salt formation e.g. hydrochloric acid, hydrogen bromide, sulfuric acid, phosphoric acid, lactic acid, citric acid, tartaric acid, fumaric acid, maleic acid, succinic acid, benzenesulfonic acid, p-toluenesulfonic acid etc. can be used. (1R,25,4R)-(-)-2-[N,N-(dimethylaminoethoxy)]-2-phenyl-1,7,7-trimethylbicyclo[2.2.1]heptane of the Formula I can be particularly advantageously used in the form of the fumarate i.e. as (1R,2S,4R)-(-)-2-[N,N-(dimethylaminoethoxy)]-2-phenyl-1,7,7-trimethylbicyclo[2.2.1]heptane-2-(E)-butenedioate (1:1).

The pharmaceutical composition according to the present invention can be prepared in galenic forms generally used in pharmaceutical industry. The compositions may be solid or liquid (e.g. tablets, coated tablets, dragées, capsules or solutions). The pharmaceutical compositions may be administered orally or parenterally, preferably orally. The combined pharmaceutical compositions according to the present invention can be prepared by procedures of pharmaceutical industry known *per se.*

According to a further aspect of the present invention there is provided a process for the preparation of combined analgesic pharmaceutical compositions which comprises admixing as component A) (1R,2S,4R)-(-)-2-[N,N-(dimethylaminoethoxy)]-2-phenyl-1,7,7-trimethylbicyclo[2.2.1]heptane or a pharmaceutically acceptable acid addition salt thereof and as component B) morphine, an opioide type analgesic and/or a non-opioide type analgesic as defined above with inert pharmaceutically acceptable carriers and/or auxiliary agents and bringing the mixture into a galenic form.

According to a still further aspect of the present invention there is provided the use of a combinations as defined above for the preparation of a medicament for the alleviation of pain.

According to a still further aspect of the present invention there is provided the use of a combinations as defined above for the preparation of analgesic pharmaceutical compositions.

The potentiation of the analgesic effect of morphine by deramciclane was demonstrated on hot plate test in mice. A modified of method of Eddy et al. (Eddy, N. B., Leimback, D., Synthetic Analgasics II. Dithienylbutenyl and Dithienylbutylamines. J. Pharmacol. Exp. Ther. 107: p. 385-393, 1953) was used. Male NMRI mice (20-25 g bodyweight) were dropped on a hot plate (56±0,5 °C) and the latency time elapsed until licking the forepaws was measured. The reaction time was tested twice before treatment. Animals were discarded if the first basal latency time >5 sec. or the difference between the two control measurements was greater than 3 sec. Mice were treated either with saline or with morphine HCl 1 mg/kg subcutanously and at the same time either with vehicle or with deramciclane or buspirone, HCl 30 mg/kg intraperitoneally, respectively. After the treatment (15, 30, 45 and 60 min.) the reaction time was measured again. Animals were regarded as positive if they produced a 2.5-fold reaction time increase at least twice compared to their first control values. Statistical analysis was performed using the chi-square method. The results are shown in Table 1.

**Table 1**

| **The influences of deramciclane and buspiron on morphine analgesia in hot plate test in mice** | | |
|---|---|---|
| Treatment | Positive/treated animals | Effect % |
| Saline sc. + 0.4 % methyl cellulose ip. | 0/10 | 0 |
| Saline sc. + Deramciclane - 30 mg/kg ip. | 0/10 | 0 |
| Morphine HCl - 1 mg/kg sc. + 0.4 % methyl cellulose ip. | 0/10 | 0 |
| Morphine HCl - 1 mg/kg sc. + Deramciclane - 30 mg/kg ip. | 6/10* | 60 |
| Saline sc. + Saline ip. | 0/10 | 0 |
| Saline sc. + Buspiron HCl - 30 mg/kg ip. | 0/10 | 0 |
| Morphine HCl - 1 mg/kg sc. + Saline ip. | 0/10 | 0 |
| Morphine HCl - 1 mg/kg sc. + Buspiron HCl - 30 mg/kg ip. | 1/10 | 10 |

| | | |
|---|---|---|
| * p<0,05 | | |

Neither morphine HCl 1 mg/lcg sc. nor deramciclane 30 mg/kg ip. nor buspiron HCl 3 0 mg/kg ip. alone produced analgesia in this test. Surprisingly, if the ineffective doses of morphine HCl (1 mg/kg sc.) and deramciclane (30 mg/kg ip.) were administered at the same time then the analgesic effect of this combination was statistically significant. The anxiolytic buspiron did not influence the analgesic effect of morphine.

These results prove that the potentiation of the analgesic effect of morphine by deramciclane is surprising because it does not follow from its anxiolytic effect. This is also emphasized by the experimentally certified recognition according to which neither deramciclane nor buspiron possess analgesic effect in itself.

## Claims

1. Combined analgesic pharmaceutical composition which comprises as component A)
(1R,2S,4R)-(-)-2-[N,N-(dimethylaminoethoxy)]-2--phenyl-1,7,7-trimethylbicyclo[2.2.1]heptane of the Formula 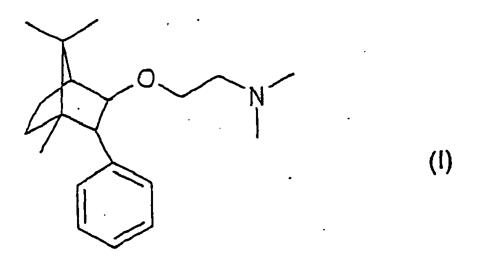 or a pharmaceutically acceptable acid addition salt thereof and as component B) morphine, an opioide type analgesic, namely tramadol, codeine, dihydrocodeine, nalbufine or buprenorfine or a pharmaceutically acceptable salt thereof, comprising component A) and the last-named component B) in a weight ratio of 500:1, preferably 100:1, particularly preferably 30:1, and/or a non-opioide type analgesic, namely acetyl salicylic acid, paracetamol, aminophenazone, diclofenac, naproxen, ibuprofene, piroxicam or a pharmaceutically acceptable salt thereof, comprising component A) and the last-named component B) in a weight ratio of 1:500, preferably 1:100, particularly preferably 1:30, and comprising component A) and component B) for a daily dosage of 0.1 to 150 mg/kg, in admixture with suitable pharmaceutical carriers and/or auxiliary agents.

2. Combined pharmaceutical composition according to Claim 1 comprising as component A)
(1R,2S,4R)-(-)-2-[N,N-(dimethylaminoethoxy)]-2--phenyl-1,7,7-trimethylbicyclo[2.2.1]heptane-2-(E)--butenedioate (1:1).

3. Combined pharmaceutical composition according to Claim 1 comprising as component A)
(1R,2S,4R)-(-)-2-[N,N-(dimethylaminoethoxy)]-2--phenyl-1,7,7-trimethylbicyclo[2.2.1]heptane or a pharmaceutically acceptable acid addition salt thereof which contains not more than 0.2% of (1R,3S,4R)-(-)-3-[2-N,N-(dimethylaminoethyl)]-1,7,7--trimethylbicyclo[2.2.1]heptane-2-one of the Formula 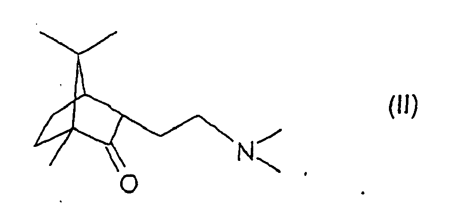 or a pharmaceutically acceptable acid addition salt thereof.

4. Combined pharmaceutical composition according to Claim 3 comprising as component A)
(1R,2S,4R)-(-)-2-[N,N-(dimethylaminoethoxy)]-2--phenyl-1,7,7-trimethylbicyclo[2.2.1]heptane-2-(E)--butenedioate (1:1) which contains not more than 0.2% of (1R,3S,4R)-(-)-3-[2-N,N-(dimethylaminoethyl)]--1,7,7-trimethylbicyclo[2.2.1]heptane-2-one-2-(E)--butenedioate (1:1).

5. Process for the preparation of the combined analgesic pharmaceutical compositions of claims 1 to 4 which comprises admixing as component A)
(1R,2S,4R)-(-)-2-[N,N-(dimethylaminoethoxy)]-2--phenyl-1,7,7-trimethylbicyclo[2.2.1]heptane or a pharmaceutically acceptable acid addition salt thereof and as component B) the morphine and/or the opioide-type analgesic tramadol, codeine, dihydrocodeine, nalbufine or buprenorfine or a pharmaceutically acceptable salt thereof and/or the non-opioide type analgesic acetyl salicylic acid, paracetamol, aminophenazone, diclofenac, naproxen, ibuprofene, piroxicam or a pharmaceutically acceptable salt thereof, with inert pharmaceutically acceptable carriers and/or auxiliary agents and bringing the mixture into a galenic form.

6. Use of a combination comprising as component A)
(1R, 2S, 4R)-(-)-2-[N,N-(dimethylaminoethoxy)]-2--phenyl-1,7,7-trimethylbicyclo[2.2.1]heptane or a pharmaceutically acceptable acid addition salt thereof and as component B) morphine and/or the opioide-type analgesic tramadol, codeine, dihydrocodeine, nalbufine or buprenorfine or a pharmaceutically acceptable salt thereof, and/or the non-opioide type analgesic acetyl salicylic acid, paracetamol, aminophenazone, diclofenac, naproxen, ibuprofene, piroxicam or a pharmaceutically acceptable salt thereof, according to claims 1 to 4 for preparing a medicament for the alleviation of pain.

7. Use of a combination comprising as component A)
(1R, 2S, 4R)-(-)-2-[N,N-(dimethylaminoethoxy)]-2--phenyl-1,7,7-trimethylbicyclo[2.2.1]heptane or a pharmaceutically acceptable acid addition salt thereof and as component B) morphine and/or the opioide-type analgesic tramadol, codeine, dihydrocodeine, nalbufine or buprenorfine or a pharmaceutically acceptable salt thereof, and/or the non-opioide type analgesic acetyl salicylic acid, paracetamol, aminophenazone, diclofenac, naproxen, ibuprofene, piroxicam or a pharmaceutically acceptable salt thereof, according to claims 1 to 4 for the preparation of analgesic pharmaceutical compositions.

## Patentansprüche

1. Kombinierte analgetische pharmazeutische Zusammensetzung, welche als Komponente A) (1R, 2S, 4R)-(-)-2-[N,N-(Dimethylaminoethoxy)]-2-phenyl-1,7,7-trimethylbicyclo-[2.2.1]-heptan der Formel 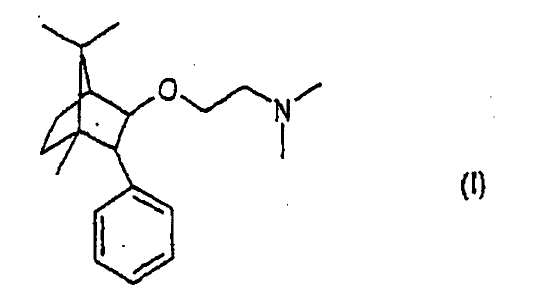 oder ein pharmazeutisch annehmbares Säureadditionssalz davon und als Komponente B) Morphin, ein Schmerzmittel vom Opioid-Typ, nämlich Tramadol, Codein, Dihydrocodein, Nalbufin oder Buprenorfin oder ein pharmazeutisch annehmbares Salz davon, umfasst, umfassend die Komponente A) und die zuletzt genannte Komponente B) in einem Gewichtsverhältnis von 500 : 1, vorzugsweise 100 : 1, besonders bevorzugt von 30 : 1, und/oder ein Schmerzmittel vom nicht-Opioid-Typ, nämlich Acetylsalicylsäure, Parcetamol, Aminophenazon, Diclofenac, Naproxen, Ibuprofen, Piroxicam oder ein pharmazeutisch annehmbares Salz davon, umfassend die Komponente A) und die zuletzt genannte Komponente B) in einem Gewichtsverhältnis von 1 : 500, vorzugsweise von 1 : 100, besonders bevorzugt von 1 : 30, und umfassend die Komponente A) und die Komponente B) für eine Tagesdosis von 0,1 bis 150 mg/kg in Vermischung mit geeigneten pharmazeutischen Trägem und/oder Hilfsmitteln.

2. Kombinierte pharmazeutische: Zusammensetzung gemäß Anspruch 1, umfassend als Komponente A)
(1R, 2S, 4R)-(-)-2-[N,N-(Dimethylaminoethoxy)]-2-phenyl-1,7,7-trimethylbicyclo-[2.2.1]-heptan-2-(E)-butendioat(1 : 1).

3. Kombinierte pharmazeutische: Zusammensetzung gemäß Anspruch 1, umfassend als Komponente A)
(1R, 2S, 4R)-(-)-2-[N,N-(Dimethylaminoethoxy)]-2-phenyl-1,7,7-trimethylbicyclo-[2.2.1]-heptan oder ein pharmazeutisch annehmbares Säureadditionssalz davon, welches nicht mehr als 0,2 % an
(1R, 3S, 4R)-(-)-3-[2-N,N-(Dimethylaminoethyl)]-1,7,7-trimethylbicyclo-[2.2.1]-heptan-2-on der Formel 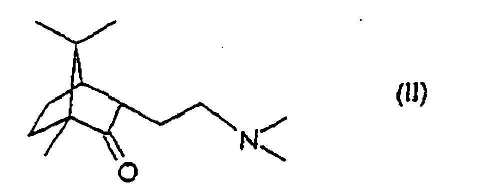 oder ein pharmazeutisch annehmbares Säureadditionssalz davon enthält.

4. Kombinierte pharmazeutische Zusammensetzung gemäß Anspruch 3, umfassend als Komponente A)
(1R, 2S, 4R)-(-)-2-[N,N-(Dimethylaminoethoxy)]-2-phenyl-1,7,7-trimethylbicyclo-[2.2.1]-heptan-2-(E) - butendioat (1 : 1), welche nicht mehr als 0,2 % an (1R, 3 S, 4R)-(-)-3-[2-N,N-(Dimethylaminoethyl)]-1,7,7-trimethylbicyclo-[2.2.1]-heptan-2-on-2-(E) - butendioat (1 : 1) enthält.

5. Verfahren zur Herstellung der kombinierten analgetischen pharmazeutischen Zusammensetzungen von Anspruch 1 bis 4, welches das Vermischen als Komponente A) von (1R, 2S, 4R)-(-)-2-[N,N-(Dimethylaminoethoxy)]-2-phenyl-1,7,7-trimethylbicyclo-[2.2.1]-heptan oder einem pharmazeutisch annehmbaren Säureadditionssalz davon und als Komponente B) von Morphin und/oder Analgetikum vom Opioid-Typ Tramadol, Codein, Dihydrocodein, Nalbufin oder Buprenorfin oder einem pharmazeutisch annehmbaren Salz davon und/oder Analgetikum vom Nicht-Opioid-Typ Acetylsalicylsäure, Paracetamol, Aminophenazon, Diclofenac, Naproxen, Ibuprofen, Piroxicam oder einem pharmazeutisch annehmbaren Salz davon mit inerten pharmazeutisch annehmbaren Trägern und/oder Hilfsmitteln und das Bringen der Mischung in eine galenische Form umfasst.

6. Verwendung einer Kombination, umfassend als Komponente A) (1R, 2S, 4R)-(-)-2-[N, N-(Dimethylaminoethoxy)]-2-phenyl-1,7,7-trimethylbicyclo-[2.2.1]-heptan oder ein pharmazeutisch annehmbares Säureadditionssalz davon und als Komponente B) Morphin und/oder das Analgetikum vom Opioid-Typ Tramadol, Codein, Dihydrocodein, Nalbufin oder Buprenorfin oder ein pharmazeutisch annehmbares Salz davon und/oder das Analgetikum vom Nicht-Opioid-Typ Acetylsalicylsäure, Paracetamol, Aminophenazon, Diclofenac, Naproxen, Ibuprofen, Piroxicam oder ein pharmazeutisch annehmbares Salz davon, gemäß den Ansprüchen 1 bis 4 zur Herstellung eines Medikamentes zur Linderung von Schmerzen.

7. Verwendung einer Kombination, umfassend als Komponente A) (1R, 2S, 4R)-(-)-2-[N, N-(Dimethylaminoethoxy)]-2-phenyl-1,7,7-trimethylbicyclo-[2.2.1]-heptan oder ein pharmazeutisch annehmbares Säureadditionssalz davon und als Komponente B) Morphin und/oder das Analgetikum vom Opioid-Typ Tramadol, Codein, Dihydrocodein, Nalbufm oder Buprenorfin oder ein pharmazeutisch annehmbares Salz davon und/oder das Analgetikum vom Nicht-Opioid-Typ Acetylsalicylsäure, Paracetamol, Aminophenazon, Diclofenac, Naproxen, Ibuprofen, Piroxicam oder ein pharmazeutisch annehmbares Salz davon, gemäß den Ansprüchen 1 bis 4 zur Herstellung von analgetischen pharmazeutischen Zusammensetzungen.

## Revendications

1. Composition pharmaceutique analgésique combinée comprenant comme composant A)
(1R, 2S, 4R)-(-)-2-[N,N-(dimethylamino-ethoxy)]-2-phényl-1,7,7-triméthylbicyclo [2.2.1] heptane de Formule 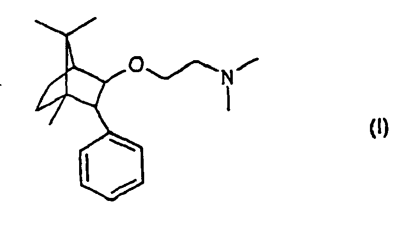 ou un sel d'addition acide pharmaceutiquement acceptable de ceux-ci et comme composant B) de la morphine, un analgésique de type opioïde, c'est-à-dire tramadol, codéine, dihydrocodéine, nalbufine ou buprenorphine, ou un sel pharmaceutiquement acceptable de ceux-ci, comprenant le composant A) et le composant B) nommé en dernier dans un rapport de poids de 500 :1, de préférence 100 :1, en particulier de préférence 30 :1, et/ou un analgésique de type non-opioïde, c'est-à-dire un acide acétyl-salicylique, paracétamol, aminophénazone, diclofénac, naproxène, ibuprofène, piroxicam ou un sel pharmaceutiquement acceptable de ceux-ci, comprenant le composant A) et le composant B) nommé en dernier dans un rapport de poids de 1 :500, de préférence 1 :100, en particulier de préférence 1 :30, et comprenant le composant A) et le composant B) pour un dosage quotidien de 0,1 à 150 mg/kg, en mélange avec des véhicules pharmaceutiques acceptables et/ou des agents auxiliaires.

2. Composition pharmaceutique combinée selon la Revendication 1 comprenant comme composant A)
(1R, 2S, 4R)-(-)-2-[N,N-(dimethylamino-ethoxy)]-2-phényl-1,7,7-triméthylbicyclo[2.2.1]heptane-2-(E)-butènedioate(1 : 1).

3. Composition pharmaceutique combinée selon la Revendication 1 comprenant comme composant A)
(1R, 2S, 4R)-(-)-2-[N,N-(dimethylamino-ethoxy)]-2-phényl-1,7,7-triméthylbicyclo [2.2.1] heptane ou un sel d'addition acide pharmaceutiquement acceptable de ceux-ci qui ne contient pas plus de 0,2% de
(1R, 3S, 4R)-(-)-3-[2-N,N-(dimethylamino-éthyl)]-1,7,7-triméthylbicyclo[2.2.1] heptane - 2 - one de Formule 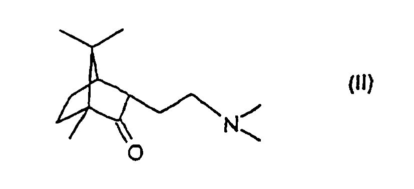 ou un sel d'addition acide pharmaceutiquement acceptable de ceux-ci.

4. Composition pharmaceutique combinée selon la Revendication 3 comprenant comme composant A)
(1R, 2S, 4R)-(-)-2-[N,N-(dimethylamino-ethoxy)]-2-phényl-1,7,7-triméthylbicyclo [2.2.1] heptane -2-(E) -- butènedioate (1 :1) qui ne contient pas plus de 0,2% de (1R, 3S, 4R)-(-)-3-[2-N,N-(diméthylamino-éthyl)] -1,7,7 - triméthylbicyclo [2.2.1]heptane-2-one-2-(E)-butènedioate (1 : 1).

5. Procédé pour la préparation de compositions pharmaceutiques analgésiques combinées des revendications 1 à 4 qui comprend en mélange comme composant A)
(1R, 2S, 4R)-(-)-2-[N,N-(dimethylamino-ethoxy)]-2-phényl-1,7,7-triméthylbicyclo [2.2.1] heptane ou un sel d'addition acide pharmaceutiquement acceptable de ceux-ci et comme composant B) de la morphine et /ou l'analgésique de type opioïde, tramadol, codéine, dihydrocodéine, nalbufine ou buprenorphine ou un sel pharmaceutiquement acceptable de ceux-ci et/ou l'analgésique de type non-opioïde, un acide acétyl-salicylique, paracétamol, aminophénazone, diclofénac, naproxène, ibuprofène, piroxicam ou un sel pharmaceutiquement acceptable de ceux-ci, avec des véhicules pharmaceutiquement acceptables inertes et/ou des agents auxiliaires et en portant le mélange sous forme galénique.

6. Utilisation d'une combinaison comprenant comme composant A)
(1R, 2S, 4R)-(-)-2-[N,N-(dimethylamino-ethoxy)]-2-phényl-1,7,7-triméthylbicyclo [2.2.1] heptane ou un sel d'addition acide pharmaceutiquement acceptable de ceux-ci et comme composant B) de la morphine et /ou l'analgésique de type opioïde, tramadol, codéine, dihydrocodéine, nalbufine ou buprenorphine ou un sel pharmaceutiquement acceptable de ceux-ci, et/ou l'analgésique de type non-opioïde, un acide acétyl-salicylique, paracétamol, aminophénazone, diclofénac, naproxène, ibuprofène, piroxicam ou un sel pharmaceutiquement acceptable de ceux-ci, selon les revendications 1 à 4 pour la préparation d'un médicament destiné à atténuer la douleur.

7. Utilisation d'une combinaison comprenant comme composant A)
(1R, 2S, 4R) - (-) - 2 - [N,N - (dimethylamino-ethoxy)] - 2 - phényl -1,7,7 - triméthylbicyclo [2.2.1] heptane ou un sel d'addition acide pharmaceutiquement acceptable de ceux-ci et comme composant B) de la morphine et /ou l'analgésique de type opioïde, tramadol, codéine, dihydrocodéine, nalbufine ou buprenorphine ou un sel pharmaceutiquement acceptable de ceux-ci, et/ou l'analgésique de type non-opioïde, un acide acétyl-salicylique, paracétamol, aminophénazone, diclofénac, naproxène, ibuprofène, piroxicam ou un sel pharmaceutiquement acceptable de ceux-ci, selon les revendications 1 à 4 pour la préparation de compositions pharmaceutiques analgésiques.
